# EUROPEAN PATENT SPECIFICATION

(11) **EP 3 992 629 B1**
(45) Date of publication and mention of the grant of the patent: **01.01.2025**
(21) Application number: 20941721.1
(22) Date of filing: 25.06.2020
(51) Int. Cl.: C12N 5/09, G01N 33/50

(54) **METHOD FOR PROVIDING INFORMATION NECESSARY FOR DIAGNOSING CANCER PATIENT'S RESISTANCE TO ANTI-CANCER AGENT AND/OR RADIATION**
VERFAHREN ZUR BEREITSTELLUNG VON INFORMATIONEN FÜR DIE DIAGNOSE DER RESISTENZ VON KREBSPATIENTEN GEGENÜBER ANTIKREBSMITTEL UND/ODER BESTRAHLUNG
PROCÉDÉ DE FOURNITURE D'INFORMATIONS NÉCESSAIRES POUR DIAGNOSTIQUER LA RÉSISTANCE D'UN PATIENT ATTEINT DE CANCER À UN AGENT ET/OU À UN RAYONNEMENT ANTICANCÉREUX

(43) Date of publication of application: 04.05.2022
(73) Proprietor: Next & Bio Inc., Seoul 08826 (KR)
(72) Inventor: CHUNG, Seok, Seoul 04024 (KR); YANG, Ji Hoon, Seoul 04413 (KR); CHOI, Dong Hee, Seoul 02578 (KR); JUNG, Yong Hun, Seoul 02860 (KR)
(74) Representative: Kador & Partner Part mbB
(86) International application number: PCT/KR2020/008285
(87) International publication number: WO 2021/261625

(56) References cited:
- KR-A- 20140 113 139
- KR-A- 20170 003 177
- KR-A- 20180 115 236
- KR-A- 20180 136 410
- KR-A- 20200 081 296
- KR-A- 20210 069 173
- KR-B1- 102 237 426
- KR-B1- 102 333 275
- US-A1- 2017 253 844
- MICHEL CHAVES ET AL: "A Practical Fluorescence-Based Screening Protocol for Polyethylene Terephthalate Degrading Microorganisms", JOURNAL OF THE BRAZILIAN CHEMICAL SOCIETY, vol. 29, no. 6, 1 January 2017 (2017-01-01), Sao Paulo, BR, pages 1278 - 1285, XP055713365, ISSN: 0103-5053, DOI: 10.21577/0103-5053.20170224
- WELZEL K ET AL: "Enzymatischer Abbau von Polyester-Nanopartikeln", CHEMIE INGENIEUR TECHNIK, WILEY VCH. VERLAG, WEINHEIM; DE, vol. 74, no. 10, 9 October 2002 (2002-10-09), pages 1496 - 1500, XP071059896, ISSN: 0009-286X, DOI: 10.1002/1522-2640(20021015)74:10<1496::AID-CITE1496>3.0.CO;2-P
- BUNZEL HANS ADRIAN ET AL: "Speeding up enzyme discovery and engineering with ultrahigh-throughput methods", CURRENT OPINION IN STRUCTURAL BIOLOGY, ELSEVIER LTD, GB, vol. 48, 3 February 2018 (2018-02-03), pages 149 - 156, XP085354238, ISSN: 0959-440X, DOI: 10.1016/J.SBI.2017.12.010

## Description

### [Technical Field]

The present invention relates to a method for diagnosis of resistance to an anticancer agent and/or radiation of a subject with cancer. More specifically, the present invention relates to a method for diagnosis of resistance to an anticancer agent and/or radiation by culturing a cancer organoid from a cancer tissue obtained from a subject with cancer to select a cancer organoid having resistance to an anticancer agent and/or radiation.

### [Background Art]

An organoid is also called "an organ analog" or "organ-like" and is an organ-specific cell aggregate produced by re-aggregating and recombining cells separated from stem cells or organ-originating cells by a 3D culture method. The organoid includes specific cells of a model organ, reproduces the specific functions of the organ, and can be spatially organized in a form similar to the actual organ. It has been reported that a patient-derived tumor organoid represents the characteristics of the patient's cancer cells and tissues as they are, and can reproduce the genetic variation characteristics of the patient's cancer tissues.

Organoids can be used in the fields of cell therapy, biotissue engineering, new drug development, toxicology and precision medicine. A large amount of comparable quantitative organoids and analytical methods thereof are required to enhance the utilization of organoids. However, there has been no method of quantitatively culturing organoids to date. The reason is that after Matrigel^{®}, which is the most important of the elements that grow organoids, is solidified in a dome-shape on the bottom, the organoids are grown therein, such that the organoids grow separately.

Further, since the organoids so grown in Matrigel^{®} can be grown while overlapping in a 3D support, the limits are clear.

In addition, recently, high-throughput screening techniques have been developed, which combine more stable and physiological patient-derived organoids for use in early drug discovery programs and toxicity screening.

Korean Patent No. 10-1756901 (Patent Document 1) discloses a cell culture chip capable of culturing 3D tissue cells. In the cell culture chip of Patent Document 1, a first culture part, a second culture part, and the third culture part are formed in each layer, and the degree of cell growth progress can be confirmed in each layer. However, the cell culture chip of Patent Document 1 has a problem in that organoids cannot be obtained in high yield.

Further, there is a case where the pipetting work of replacing a culture solution during cell culture is performed, and in the case of a Corning spheroid microplate capable of 3D cell culture, spheroids or organoids in cell culture are affected, so that there is a problem which is not good for the cell culture environment because there is a case where the spheroids or organoids are sucked up or the positions thereof are changed during the pipetting work.

For a cell of existing drug screening targeting solid cancers, a simple specimen in which the cells of most patients have been isolated and made in a spheroid form has been used. Such spheroid types cannot adequately simulate the patient's condition and cannot reflect the structural and biological characteristics that may cause drug resistance. In addition, although screening using organoids has begun, the screening is currently limited to drugs only, and there is no *in vitro* platform for radiation therapy. To date, no platform model that classifies cancer organoids having resistance to drugs and radiation has been developed, and no platform that can select resistant organoids has been developed.

Thus, the present inventors have conducted continuous studies on a cancer organoid having resistance to anticancer agents and/or radiation, thereby completing the present invention.

### [Related Art Documents]

### [Patent Document]

1. Korean Patent No. 10-1756901

### [Disclosure]

### [Technical Problem]

An object of the present invention is to provide information necessary for diagnosis of resistance to an anticancer agent and/or radiation of a subject with cancer by selecting a cancer organoid having resistance to an anticancer agent and/or radiation.

Another object of the present invention is to provide a method of screening a drug by selecting a cancer organoid having resistance to an anticancer agent and/or radiation.

### [Technical Solution]

To achieve the objects, the present invention provides a method for diagnosis of resistance to an anticancer agent and/or radiation of a subject with cancer, the method including:
providing an isolated cancer tissue from a subject with cancer;
culturing the cancer tissue into a cancer organoid in a 3D cell culture plate:
   treating the cultured cancer organoid with an anticancer agent and/or radiation; and
   selecting the cancer organoid treated with the anticancer agent and/or radiation as a cancer organoid having resistance to an anticancer agent and/or radiation when the cancer organoid treated with the anticancer agent and/or radiation has a viability equal to or more than that before the treatment with the anticancer agent and/or radiation,
   wherein in the culturing of the organoid, the 3D cell culture plate includes 0 to 2 vol% of an extracellular matrix-based hydrogel, and
   wherein the 3D cell culture plate includes:
      a well plate including a plurality of main wells and a plurality of sub wells formed at lower portions of the main wells to be injected with a cell culture solution and including recessed parts on a bottom surface thereof; and a connector for large-capacity and high-speed high content screening (HCS), which supports the well plate, and
      the connector for high content screening (HCS) includes a base equipped with a fixing means so as to be attached to and detached from a lower end of the well plate and a cover positioned on an upper portion of the well plate to be coupled to the base, the main well has a step formed so as to be tapered at a predetermined site, and the step has an inclination angle (θ) ranging from 10 to 60° with respect to a wall of the main well.

The subject with the cancer may be a mammal, and is preferably a human.

The "cancer organoid having resistance to an anticancer agent and/or radiation" means an organoid which is hard to kill even when treated with an anticancer agent and/or radiation, and includes i) a cancer organoid having resistance to an anticancer agent, ii) a cancer organoid having resistance to radiation and iii) a cancer organoid having resistance to both an anticancer agent and radiation.

**In** the selecting of the cancer organoid treated with the anticancer agent and/or radiation as a cancer organoid having resistance to an anticancer agent and/or radiation, "when the cancer organoid treated with the anticancer agent and/or radiation has a viability equal to or more than that" means the case where a cancer organoid treated with an anticancer agent and/or radiation has the same viability as or a viability which is not less than an organoid before treatment with an anticancer agent and/or radiation. Specifically, it refers to the case where the cancer organoid treated with the anticancer drug and/or radiation has a viability ranging from 100 to 70% compared to an organoid before treatment with an anticancer agent and/or radiation. Alternatively, it refers to the case where the cancer organoid treated with the anticancer drug and/or radiation has a size which is not reduced by 30% to 60% or more compared to an organoid before treatment with an anticancer agent and/or radiation. The fact that the size of cancer organoid is significantly reduced means that there was an anticancer therapeutic effect, but it also means that if the size is not significantly reduced, the cancer organoid has resistance.

**In** the selecting of the cancer organoid treated with the anticancer agent and/or radiation as a cancer organoid having resistance to an anticancer agent and/or radiation, the cancer organoid may be selected as a cancer organoid having resistance to an anticancer agent and/or radiation when the cancer organoid treated with the anticancer agent and/or radiation has a size which is not reduced by 30% to 60% or more compared to an organoid before treatment with an anticancer agent and/or radiation according to the concentration of the anticancer agent and the degree of radiation irradiation.

The present invention produced a standard-type cancer organoid from a cancer tissue of a subject with cancer using a method of producing a standard-type cancer organoid. Next, when the cancer organoid treated with the anticancer agent and/or radiation has a size which is not reduced compared to an organoid before treatment with an anticancer agent and/or radiation, it was selected as a cancer organoid having resistance to an anticancer agent and/or radiation. And, the subject with cancer, who is the main character of the cancer organoid having resistance to an anticancer agent and/or radiation selected in this manner was selected as a subject having resistance to an anticancer agent and/or radiation.

Specifically, a cancer organoid having a size which is not reduced only during treatment with an anticancer agent is a cancer organoid having resistance to an anticancer agent (therefore, a subject of this cancer organoid is determined as a subject with cancer, who has resistance to an anticancer agent), a cancer organoid having a size which is not reduced only during a treatment with radiation is a cancer organoid having resistance to radiation (therefore, a subject of this cancer organoid is determined as a subject with cancer, who has resistance to radiation), and a cancer organoid having a size which is not reduced in treatment with both an anticancer agent and radiation is a cancer organoid having resistance to both anticancer agent and radiation (therefore, a subject of this organoid is determined as a subject with cancer, who has resistance to an anticancer agent and radiation).

In the culturing of the cancer organoid, the extracellular matrix-based hydrogel is added at 0 to 2 vol%.

The extracellular matrix-based hydrogel may be Matrigel^{®} (product trade name).

A size of the organoids may be 300 to 500 µm in diameter.

The cancer may be colorectal cancer, lung cancer, gastric cancer, skin cancer, prostate cancer, breast cancer, cervical cancer, thyroid cancer, fibrosarcoma, uterine sarcoma, ovarian cancer, pancreatic cancer or hematological cancer, but is not limited thereto.

The anticancer agent may be one or more selected from the group consisting of a cytotoxic anticancer agent, a targeted therapeutic agent, an immunotherapeutic agent, and a metabolic anticancer agent. More specifically, it may be selected from the group consisting of 5-fluorouracil (5-FU), doxifluridine, oxaliplatin, cisplatin, mitoxantrone, adriamycin, doxorubicin, irinotecan, and paclitaxel, and any anticancer agent can be used as long as it is an anticancer agent known in the art.

As will be described in detail below, when the 3D cell culture plate of the present invention is used, a standard-type cancer organoid may be mass-produced.

The sub well of the 3D cell culture plate may have an inclined surface formed so as to taper toward the recessed part, the sub wells may have an upper end diameter ranging from 3.0 to 4.5 mm, the recessed parts may have an upper end diameter ranging from 0.45 to 1.5 mm, an inclined surface (θ₂) between the sub well and the recessed part may range from 40 to 50°, and a length ratio of the diameter of the sub wells to the diameter of the recessed parts may range from 1:0.1 to 0.5.

The main wells of the 3D cell culture plate may have an individual volume ranging from 100 to 300 µl, the recessed parts may have an individual volume ranging from 20 to 50 µl, and an individual volume ratio of the main well to the recessed part may be 1 : 0.1 to 0.5 on average.

The main well includes a space part between the step and the sub well, the space part may have a height (aₕ) ranging from 2.0 to 3.0 mm on average, the sub well may have a height (bₕ) from 1.0 to 2.0 mm on average, and a height ratio (aₕ:bₕ) of the space part to the sub well may range from 1:0.3 to 1.

**In** another aspect, the present invention provides a method of screening a drug which alleviates the drug resistance of cancer cells, the method including:
isolating a cancer tissue from a subject with cancer;
culturing the cancer tissue into a cancer organoid in a 3D cell culture plate:
   treating the cultured cancer organoid with an anticancer agent and/or radiation;
   selecting the cancer organoid treated with the anticancer agent and/or radiation as a cancer organoid having resistance to an anticancer agent and/or radiation when the cancer organoid treated with the anticancer agent and/or radiation has a viability ranging from 100% to 70% compared to that before the treatment with the anticancer agent and/or radiation;
   treating the organoid selected as the cancer organoid having resistance to the anticancer agent and/or radiation with a candidate material which alleviates the drug resistance of cancer cells along with a cancer resistance drug;
   comparing a cancer organoid viability of a group treated with the candidate material with a cancer organoid viability of a control untreated with the candidate material; and
   determining the candidate material as a drug for alleviating the drug resistance of cancer cells when the cancer organoid viability of the group treated with the candidate material is lower than the viability of the control,
   wherein in the culturing of the organoid, the 3D cell culture plate includes 0 to 2 vol% of an extracellular matrix-based hydrogel, and
   wherein the 3D cell culture plate includes:
      a well plate including a plurality of main wells and a plurality of sub wells formed at lower portions of the main wells to be injected with a cell culture solution and including recessed parts on a bottom surface thereof; and a connector for large-capacity and high-speed high content screening (HCS), which supports the well plate, and
      the connector for high content screening (HCS) includes a base equipped with a fixing means so as to be attached to and detached from a lower end of the well plate and a cover positioned on an upper portion of the well plate to be coupled to the base, the main well has a step formed so as to be tapered at a predetermined site, and the step has an inclination angle (θ) ranging from 10 to 60° with respect to a wall of the main well.

The description on the cancer cells, the organoid culture method, the organoid selection method, and the cell culture plate is as described above.

Hereinafter, the present invention will be described in detail.

Specific exemplary embodiments will be illustrated in the drawings and described in detail in the Detailed Description.

Singular expressions include plural expressions unless the context clearly indicates otherwise.

In the present invention, the term "include" or "have" is intended to indicate the presence of the characteristic, number, step, operation, constituent element, part or any combination thereof described in the specification, and should be understood that the presence or addition possibility of one or more other characteristics or numbers, steps, operations, constituent elements, parts or any combination thereof is not pre-excluded.

In general, when organoids are cultured, a hydrogel is used to play the role of an extracellular matrix. For example, after Matrigel^{®} is solidified in a dome-shape on the bottom of a cell culture plate, organoids are grown therein, and organoids grow in different sizes and shapes and the functions thereof develop differently for this reason, such that there is a problem in that it is difficult to standardize the organoids.

The present invention produces organoids using a 3D cell culture plate which does not include, or includes a minimal amount of extracellular matrix-based hydrogel. A specific description on the 3D cell culture plate of the present invention is as follows.

**In** an exemplary embodiment, the present invention uses a 3D cell culture plate including:
a well plate including a plurality of main wells and a plurality of sub wells formed at lower portions of the main wells to be injected with a cell culture solution and including recessed parts on a bottom surface thereof; and
a connector for large-capacity and high-speed high content screening (HCS), which supports the well plate,
wherein the connector for high content screening (HCS) includes a base equipped with a fixing means so as to be attached to and detached from a lower end of the well plate and a cover positioned on an upper portion of the well plate to be coupled to the base,
the main well has a step formed so as to be tapered at a predetermined site, and the step has an inclination angle (θ) ranging from 10 to 60° with respect to a wall of the main well.

A 96-well plate in the related art has a problem in that it takes a lot of time and costs because experiments and analyses should be performed several times or more in order to evaluate the efficacy of a drug in high yield. Furthermore, there is a case where the pipetting work of replacing a culture solution during cell culture is often performed, and in the case of a Corning spheroid microplate in the related art, spheroids or organoids in cell culture are affected, so that there was a problem which is not good for the cell culture environment because there is a case where spheroids or organoids are sucked up or the positions thereof are changed during the pipetting work.

Therefore, the present invention has been made in an effort to solve the above-described problems, and provides a cell culture plate capable of manufacturing spheroids/organoids in high yield by including a plurality of sub wells in a plurality of main wells in a well plate, and capable of uniformly capturing images in the well plate by including a connector for large-capacity high-speed high content screening (HCS), which supports the well plate to reduce a tolerance when a large-capacity and high-speed image is captured. Furthermore, the present invention provides a cell culture plate capable of minimizing the effects of the pipetting work during replacement of a medium on cells to be cultured by the step of the main well.

Hereinafter, preferred exemplary embodiments of the present invention will be described in detail with reference to the accompanying drawings.

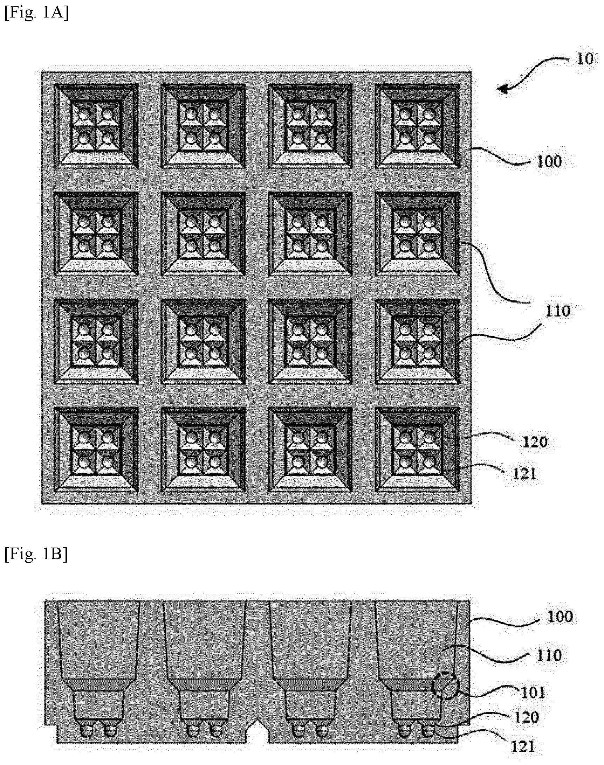

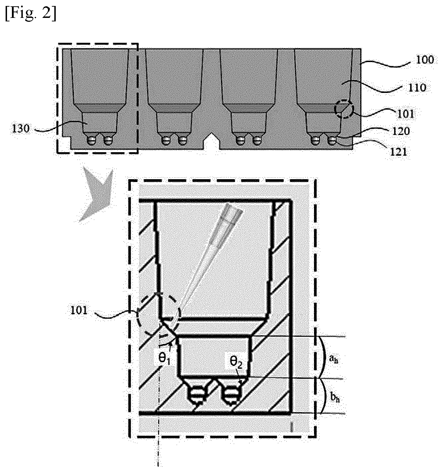

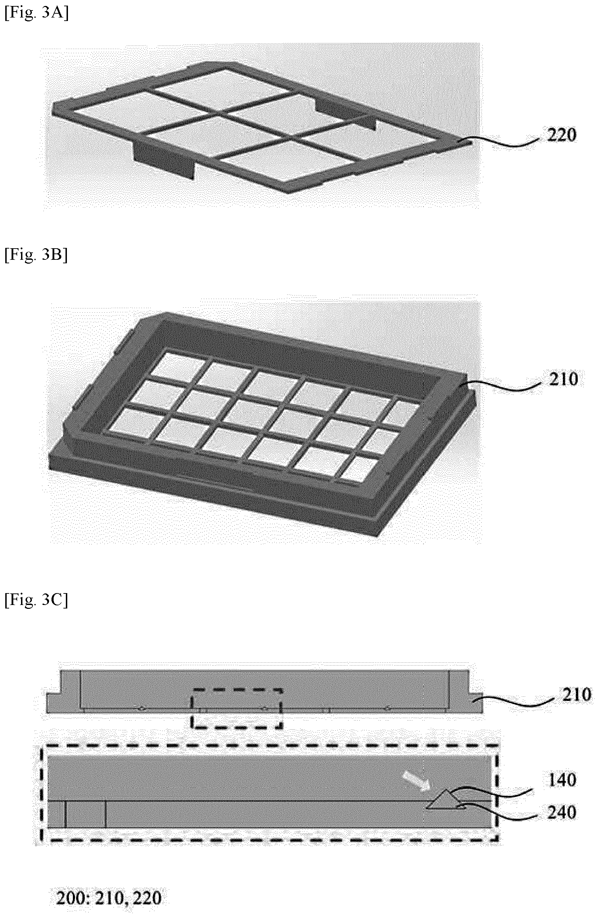

FIG 1A is a front view of a cell culture plate according to an exemplary embodiment of the present invention , FIG 1B is a cross-sectional view of the cell culture plate according to an exemplary embodiment of the present invention, FIG 2 is a view illustrating a main well formed in the cell culture plate according to an exemplary embodiment of the present invention, and FIG 3 is a view illustrating a well plate, a base and a cover of the cell culture plate according to an exemplary embodiment of the present invention ((A) a cover, (B) a base, and (C) a fixing means of a microplate and a base).

Hereinafter, a cell culture plate according to an exemplary embodiment of the present invention will be described in detail with reference to FIGS. 1 to 3.

As illustrated in FIGS. 1 to 3, a cell culture plate 10 according to an exemplary embodiment of the present invention includes a well plate 100 including a plurality of main wells 110 and a plurality of sub wells 120 formed at lower portions of the main wells 110 to be injected with a cell culture solution and including recessed parts 121 on a bottom surface thereof; and a connector 200 for large-capacity and high-speed high content screening (HCS), which supports the well plate 100.

First, the well plate 100 according to an exemplary embodiment of the present invention will be described in detail.

The well plate 100 is made into a plate shape that is plastic injection-molded through a mold. In order to manufacture a mold for plastic injection as described above, the main well 110 has a repeating pattern as a well structure such that the unit cost of production can be reduced and the size can be easily increased using fine machining. Therefore, cells are easily mass-produced, and the cells can be transformed into various sizes according to the user's requirements and used.

A plurality of the main wells 110 is formed in the well plate 100, and each main well 110 includes a step 101. The step 101 is formed at a predetermined site of the main well 110, and more specifically, the step 101 may be formed at a position which is 1/3 to 1/2 of a total length of the main well 110, and the step 101 may be formed at a position which is 1/3 to 1/2 of the total length from the lower end of the main well 110.

In the related art, there is a case where the pipetting work of replacing a culture solution during cell culture is performed, and in this case, spheroids or organoids in cell culture are affected, so that there is a problem which is not good for the cell culture because there is a case where the spheroids or organoids are sucked up or the positions thereof are changed during the pipetting work, but the step 101 is provided to prevent this problem.

The step 101 may be a space to which a pipette is applied, and specifically, has an inclination angle (θ) ranging from 10 to 60° with respect to a wall of the main well 110. Alternatively, the step 101 may have an inclination angle ranging from 20 to 50°, preferably ranging from 30 to 45°. When the inclination angle of the step 101 is less than 10°, the inclination angle within the main well 110 is so small that the space to which a pipette can be applied is not sufficient, and as a result, when the culture solution in the main well 110 is sucked up, the pipette may slide inside the sub well 120, causing spheroids or organoids to be sucked up, or the positions thereof, and the like to be changed. Furthermore, when the inclination angle (θ) exceeds 60°, a space to which a pipette can be applied is provided, but the inclination angle of the step 101 is so large that it may be difficult to sufficiently suck up the culture solution, and when cells are seeded in the sub well 120, a problem in that cells are seeded on the step 101 without entering all the sub wells 120 may occur. Therefore, it is desirable to have an inclination angle in the above-described range.

Meanwhile, the main well according to an exemplary embodiment of the present invention may include a space part 130 between the step 101 and a sub well 120 to be described below. Specifically, the space part 130 is a space into which a culture solution is injected, and is a space in which cells inside the sub well 120 can share the same culture solution.

More specifically, the space part 130 may have a height (aₕ) ranging from 2.0 to 3.0 mm on average, or ranging from 2.2 to 2.8 mm, or ranging from 2.3 to 2.7 mm on average. Furthermore, the sub well 120 may have a height (bₕ) ranging from 1.0 to 2.0 mm on average, or ranging from 1.2 to 1.8 mm on average.

For example, the space part 130 may have a height (aₕ) of 2.5 mm on average, and the sub well may have a height (bₕ) of 1.5 mm on average.

In this case, a height ratio (aₕ:bₕ) of the space part to the sub well 120 may range from 1:0.3 to 1, and more specifically, a height ratio (aₕ:bₕ) of the space part to the sub well 120 may be 1:0.4 to 0.9 or 1:0.5 to 0.8. When a ratio of the height of the space part to the height of the sub well 120 is less than 1:0.3, the cells in culture may escape from the inside even with a small force during the exchange of the media of the sub well 120, and when a ratio of the height of the space part to the height of the sub well 120 exceeds 1:1, the culture solution required for the cells is not sufficiently converted, so that cell death may be induced. Therefore,
it is preferred that the space part 130 and the sub well 120 have the above-described height range and height ratio.

Next, the sub wells 120 are formed at lower portion of each of the main wells 110 and include recessed parts 121 on a bottom surface thereof. As a particular aspect, the sub well 120 may include a plurality of recessed parts at lower portions of the main well 110.

The sub wells 120 included at the lower portion of the main well 110 have the same size and shape, thereby enabling organoids to be produced under uniform conditions.

The sub well 120 may have an inclined surface formed so as to taper toward the recessed part 121. Specifically, the horizontal area of the upper portion of the sub well 120 may become smaller as it descends in the vertical direction. For example, the upper portion of the sub well 120 may be formed in an inverted pyramid shape. In the illustrated exemplary embodiment, the upper portion of the sub well 120 may be formed in a shape such as a pyramid shape or a funnel shape in which the horizontal area of the upper portion of the sub well 120 becomes smaller as it descends in the vertical direction.

In particular, the cell culture plate may produce a large amount of spheroids or organoids under uniform conditions by including a plurality of the sub wells 120 so as to have the same size and shape.

As a particular aspect, one main well 110 can include 4 to 25 sub wells 120 of the same size, and the entire microplate 100 may include 96 to 1,728 sub wells 120. Accordingly, the size can be controlled in the same precise manner. Therefore, it is possible to mass-produce an organoid having a uniform size and shape, that is, a standard-type cancer organoid.

Furthermore, the sub well 120 includes a recessed part 121, and a space is formed in the lower portion of the recessed part such that 3D spheroids or organoids can be cultured in the recessed part 121. Specifically, the recessed part 121 may be in the form of the letter 'U', 'V', or ' 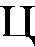 ', and for example, the recessed part 121 may be in the form of the letter 'U'.

The sub well 120 may have an upper end diameter ranging from 3.0 to 4.5 mm, or ranging from 3.5 to 4.3 mm, or 4 mm on average. Furthermore, the recessed part 121 may have an upper end diameter of 0.45 to 1.5 mm, or 0.5 to 1.0 mm or 0.5 mm on average.

Furthermore, a length ratio of the diameter of the sub well 120 to the diameter of the recessed part 121 may range from 1:0.1 to 0.5, and preferably, a length ratio of the diameter of the sub well 120 to the diameter of the recessed part 121 may be 1:0.12.

When the upper end diameter of the recessed part 121 is less than 0.1 compared to the upper end diameter 1 of the sub well 120, a cell culture space of the recessed part 121 cannot be sufficiently provided, which may cause a problem in that cells escape even with a small force during the replacement of the culture solution, and when the upper end diameter of the recessed part 121 is exceeds 0.5 compared to the upper end diameter 1 of the sub well 120, a sufficient culture solution required for cells cannot be replaced, which may cause a problem in that it is difficult to stably culture cells.

Meanwhile, an inclination surface between the sub well 120 and the recessed part 121 may have an inclination angle (θ₂) of 40 to 50°, 42 to 48°, 43 to 47°, or an inclination angle (θ₂) of 45° on average, with respect to a wall of the main well.

The above-described sub well 120 has an advantage in that cells can be cultured at 100 to 1000 cells/well or less, and the organoid size can be stably controlled when cells at 100 cells/well or less are seeded.

Further, the main well 110 according to an exemplary embodiment of the present invention has an individual volume ranging from 100 to 300 µl, the recessed part 121 has an individual volume ranging from 20 to 50 µl, and an individual volume ratio of the main well 110 to the recessed part 121 is characterized by being 1:0.07 to 0.5 on average. Preferably, the main well according to an exemplary embodiment has an individual volume ranging from 250 to 300 µl, the recessed part has an individual volume ranging from 25 to 35 µl, and an individual volume ratio of the main well 110 to the recessed part 121 may be 1:0.11 on average.

Specifically, when the main well 110 has an individual volume less than 100 µl, a problem in that a sufficient culture solution cannot be accommodated during cell culture may occur, and when the individual volume exceeds 300 µl, culture efficiency may be reduced.

Furthermore, the recessed part 121 is a space in which cells are substantially cultured, and when the volume is less than 20 µl, the cell culture space is not sufficient, which may cause a problem in that cells escape, and when the volume exceeds 50 µl, a problem in that it is difficult to stably culture cells and the like may occur. Therefore, it is preferred that the main well 110 and the recessed part 121 have volumes in the above-described ranges.

Due to the aforementioned configuration of the cell culture plate of the present invention, the organoid can be well formed even though a hydrogel (Matrigel^{®}) is not included, or is included at less than 2 vol%.

The cell culture plate 10 according to an exemplary embodiment of the present invention includes a connector 200 for large-capacity and high-speed high content screening (HCS), which supports the well plate 100. Herein, the connector 200 for large-capacity and high-speed high content screening (HCS) refers to a connector 200 which is attached to a high content screening (HCS) system, and specifically, the connector may refer to a base 210 and a cover 220 in the present invention.

More specifically, the connector for large-capacity and high-speed high content screening (HCS) includes the base 210 equipped with fixing means 140 and 240 so as to be attached to and detached from a lower end of the well plate 100 and a cover 220 positioned on an upper portion of the well plate 100 to be coupled to the base 210. Moreover, the upper end of the base 210 and the lower end of the well plate 100 are characterized by including fixing means 140 and 240 that can be fixed so as to be attached to and detached from each other.

In this case, the base includes a convex part 240 for supporting the well plate 100, and the well plate 100 may include a concave part 140 facing the convex part 240 of the base 210. The well plate 100 may be fixed by the fixing means to uniformly capture images during screening.

The base may be formed of a polyethylene, polypropylene, polystyrene, polyethylene terephthalate, polyamide, polyester, polyvinyl chloride, polyurethane, polycarbonate, polyvinylidene chloride, polytetrafluoroethylene, polyether ether ketone or polyetherimide material, but is not limited thereto.

The well plate may be formed of a polydimethylsilicone, high-fat modified silicone, methylchlorophenyl silicone, alkyl-modified silicone, methylphenylsilicone, silicone polyester, or amino-modified silicone material, but is not limited thereto.

### [Advantageous Effects]

The cancer organoid having resistance to an anticancer agent and/or radiation selected according to the present invention can be used for predicting the effective effect of anticancer treatment on a patient with cancer.

Through the present invention, when large-scale drug screening is performed, organoids having a uniform size, comparable to each other, are formed unlike the conventional organoids, so that quantitative analysis of the effects of the drug and radiation becomes possible. This makes it possible to select a drug suitable for the specificity of each variant gene, and more effective drug treatment can be achieved.

### [Description of Drawings]

FIG 1A is a front view of a cell culture plate according to an exemplary embodiment of the present invention, and FIG 1B is a cross-sectional view of the cell culture plate according to an exemplary embodiment of the present invention.
FIG 2 is a view illustrating, in detail, a main well formed in the cell culture plate according to an exemplary embodiment of the present invention.
FIG 3 is a view illustrating a well plate, a base, and a cover of the cell culture plate according to an exemplary embodiment of the present invention ((A) a cover, (B) a base, and (C) a fixing means of a microplate and a base).
FIG 4 is a view illustrating the high-speed mass imaging results of Example 1 and Comparative Example 1 ((A) Example 1, (B) Comparative Example 1).
FIG 5 illustrates the results of culturing organoids in which Matrigel^{®} is included at 2 vol% according to an exemplary embodiment of the present invention and in which Matrigel^{®} is not used.
FIG 6 illustrates the results of immunofluorescence staining of organoids including Matrigel^{®} at 2 vol% according to an exemplary embodiment of the present invention and in which Matrigel^{®} is not used.
FIG 7A is a set of photographs illustrating the high-speed mass imaging results of Example 1, and FIG 7B is a graph illustrating the area of the organoids cultured in Example 1.
FIG 8A is a set of photographs illustrating the high-speed mass imaging results of Comparative Example 1, and FIG 8B is a graph illustrating the area of the organoids cultured in Comparative Example 1.
FIG 9 illustrates imaging results (left) and an organoid size distribution (right) when colorectal cancer cells according to an exemplary embodiment of the present invention are cultured for 14 days.
FIG 10 is a set of results of selecting cancer organoids having resistance to an anticancer agent and radiation after culturing the cancer organoids according to an exemplary embodiment of the present invention.
FIG 11 is a set of live/dead images after treating cancer organoids with an anticancer agent and radiation according to an exemplary embodiment of the present invention.
FIG 12 is a set of results of measuring the viability and gene expression of organoids of cancer organoids having resistance to an anticancer agent and radiation, selected according to an exemplary embodiment of the present invention.
FIG 13 illustrates the change in size of the organoid after radiation and drug treatment according to the present invention, and illustrates the ratio of the area of each group to the control.
FIG 14 schematically illustrates that mutations of each cancer organoid are confirmed after treatment with an anticancer agent and radiation, and an effective therapeutic effect is predicted through categorization and grouping.

### [Examples]

### Example A: Confirmation of production of standard-type organoid

### 1. Production of standard-type organoid

After an existing colorectal cancer organoid was put into a 15-ml tube along with 5 ml of a culture medium and the tube was centrifuged at 200 rpm for 3 minutes, the supernatant was thoroughly removed, and the organoid was completely isolated in to single cells by performing treatment with Accutase^{®} (trade name) for 7 minutes. These single cells were seeded in sub wells of a cell culture plate at about 100 cells/well, and an organoid was produced by culturing the single cells for a total of 14 days. In this case, the culture solution is a DMEM/F12-based culture solution, and B27, N2, GlutaMAX, penicillin streptomycin, nicotinamide, N-acetyl, gastrin, A-83-01, EGF, noggin, R-spondin1, and WNT3A are included in the corresponding culture solution, and organoids were produced under culture conditions in which Matrigel^{®} was not contained or 2 vol% of Matrigel^{®} was contained.

A standard-type organoid was produced by the aforementioned method. Organoids were produced under a condition in which a 2 vol% of Matrigel^{®} was contained in the culture solution (Example 1-1) and under a condition in which Matrigel^{®} was not contained (Example 1-2).

Cells were cultured in the same manner as in Example 1-1, except that the cells were seeded in sub wells at about 200 cells/well (Example 2).

Cells were cultured in the same manner as in Example 1-1, except that the cells were seeded in sub wells at about 300 cells/well (Example 3).

Cells were cultured in the same manner as in Example 1-1, and high-speed mass imaging was performed. However, in the Comparative Example, a "96-well plate in the form of the letter 'U', which is a typically used cell culture plate, was used, and an organoid was produced by seeding cells on Matrigel^{®} (Comparative Example 1).

### 2: Measurement of size and number of organoids

The ImageJ program was used for the size analysis of the organoids. Specifically, by selecting a region of interest in a phase image and applying a threshold in the ImageJ program, a part, which was not needed, was overwritten and a part, which was not properly drawn, was drawn with black. An area to which the threshold was applied was calculated using an outer periphery.

### 3: Immunofluorescence staining method

LGR5, which is an organoid stem cell, was stained and confirmed through immunofluorescence staining. First, a standard-type organoid according to the present invention was stored in a 4% paraformaldehyde solution at room temperature for 1 hour, and then stained with PBS. Then, after the standard-type organoid was refrigerated in 15% sucrose for one day and in 30% sucrose for one day, a cryo-block was manufactured using liquid nitrogen. Using the manufactured cryo-block, the block was cut to a thickness of 10 µm, and the cut cross-section was attached to a slide glass. The slide glass was treated with 0.1% Triton^{™}X (trade name) for 10 minutes, and then washed twice with PBS. After the slide glass was stored in 3% BSA at room temperature for 1 hour, immunostaining was performed using LGR5 after washing twice with PBS2, and then measurement was performed under a fluorescence microscope by adding a mounting solution thereto.

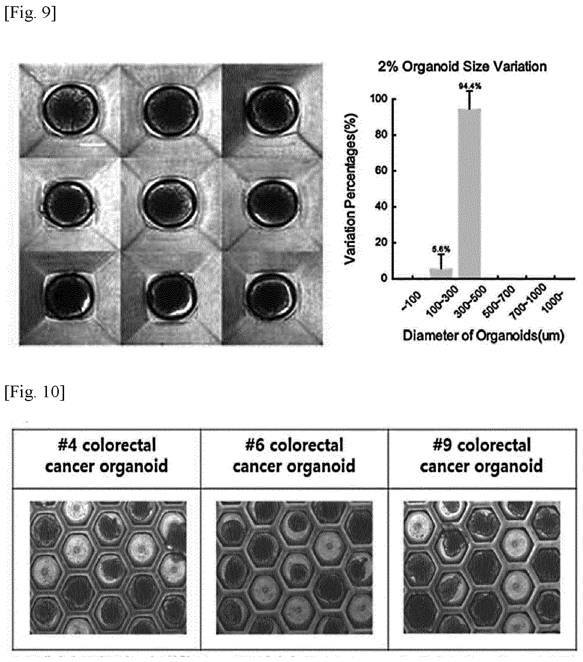

In the case of FIG 10, the cultured standard-type organoid was taken out, and Live/Dead fluorescence staining was performed. In the case of fluorescent staining, 1 mM calcein and 2 mM EtdH-1 were respectively stored at 2 µl per 1 ml and 1 µl per 1 ml in an incubator for 30 to 60 minutes, and then measurement was performed under a fluorescence microscope.

### 4. Analysis of organoid images

The cells cultured in Example 1-1 and Comparative Example 1 were photographed, and the sizes of cell spheres were compared. Spheroids were subjected to imaging by an automated plate device, and in this case, the device was allowed to perform imaging by automatically focusing. Image size analysis was performed using a macro program of the ImageJ program.

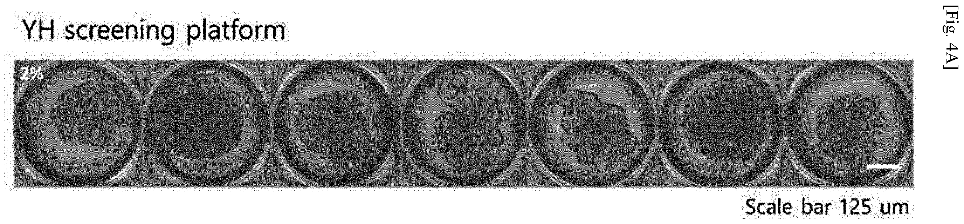

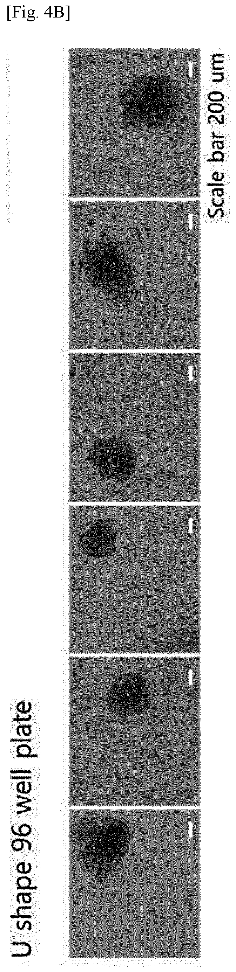

Moreover, the results are illustrated in FIG 4. FIG 4 is a view illustrating the high-speed mass imaging results of Example 1-1 and Comparative Example 1 ((A) Example 1-1, (B) Comparative Example 1).

Referring to FIG 4, it was confirmed that in the case of Example 1-1, the diameters of the cells cultured on the cell culture plate of the present invention were almost uniform. Specifically, when cells were seeded in each sub well at 100 cells/well on average, a uniform organoid that could be comparatively analyzed could be manufactured. In this case, the error range for the organoid size was around 20 µm. Through this, it can be seen that a standard organoid can be produced using the organoid culture method according to the present invention.

In contrast, it could be confirmed that in the case of Comparative Example 1 using a well plate in the related art, cell spheres having different sizes were formed. The error range of the size of organoids generated due to the growth of multiple cells in the dome morphology of one Matrigel^{®} appeared with a deviation of 150 µm or more, and the cells were grown while overlapping, such that it was impossible to perform uniform high-speed mass imaging and experiments.

The base and well plate of the present invention include a convex part and a concave part to fix each other, respectively, and the convex part and the concave part may be connected to each other for the base to firmly fix the well plate, showing that an image in the well plate can be uniformly captured.

In contrast, it can be seen that the size and shape of the organoids cultured in Comparative Example 1 are not uniform. This seems to make image analysis difficult because the focal deviation of the imaging is increased in the absence of a plate base.

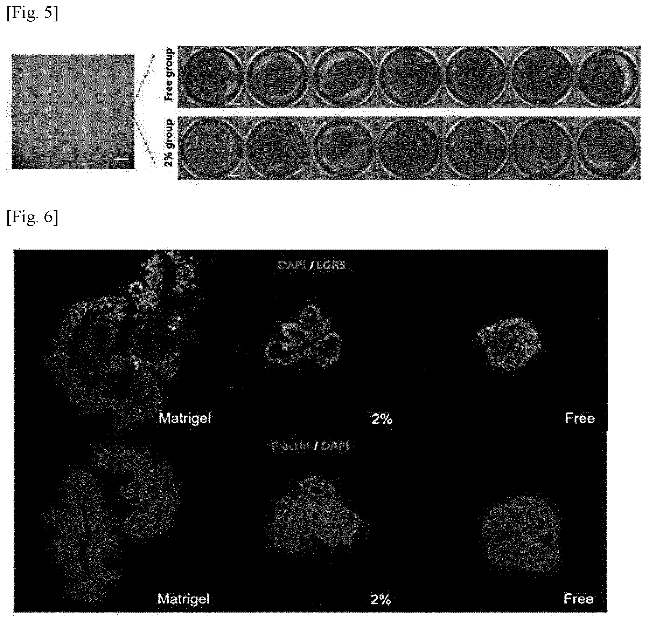

Further, referring to FIG 5, it was confirmed that when the cell culture plate of the present invention was used even without containing Matrigel^{®}, organoids were formed well.

FIG 6 confirms the expression level by staining LGR5, which is the most important marker for the formation of colorectal cancer organoids in order to confirm whether the cultured organoids were successfully formed. In addition, the presence of a colon-specific structure in the colorectal cancer organoids of a low-concentration Matrigel^{®} group or a group in which Matrigel^{®} was not used, formed through F-actin staining, was confirmed.

### 6. Production of standard-type organoid

The cells cultured in Example 1-1 and Comparative Example 1 were subjected to high-speed mass imaging.

The organoids produced in Example 1-1 and Comparative Example 1 were subjected to imaging by an automated plate device, and in this case, the device was allowed to perform imaging by automatically focusing. Image size analysis was performed using a macro program of the ImageJ program.

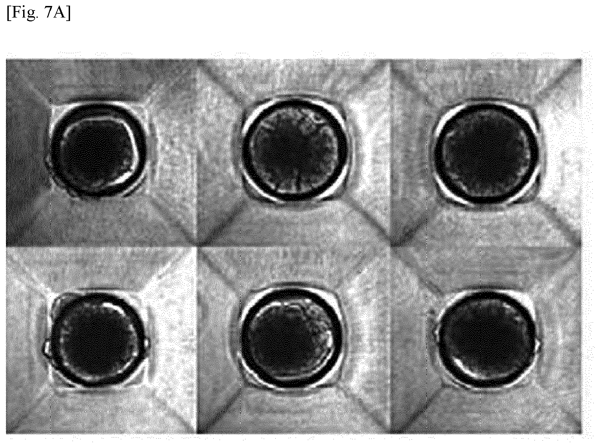

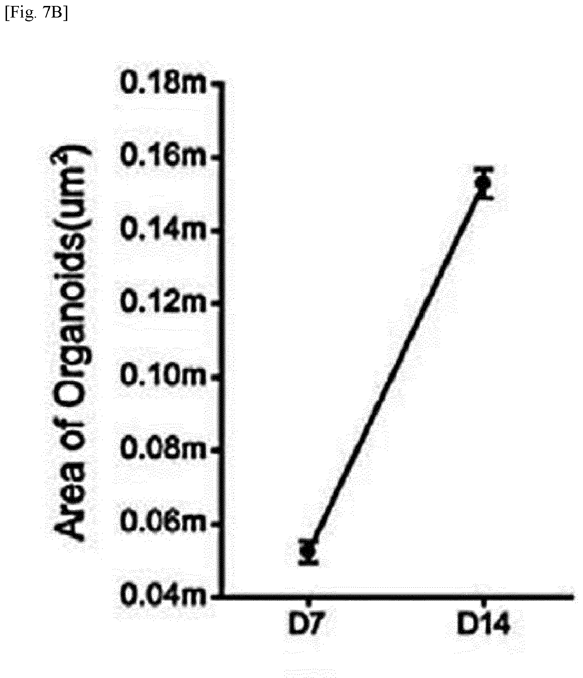

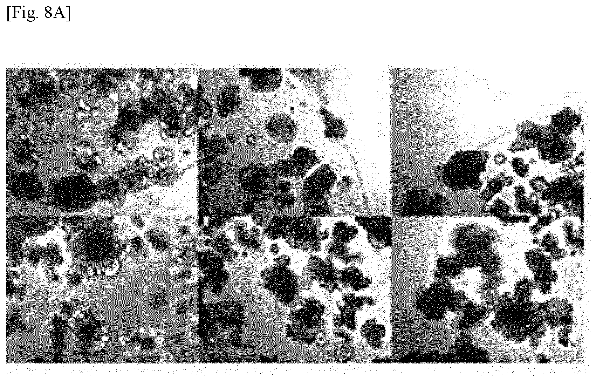

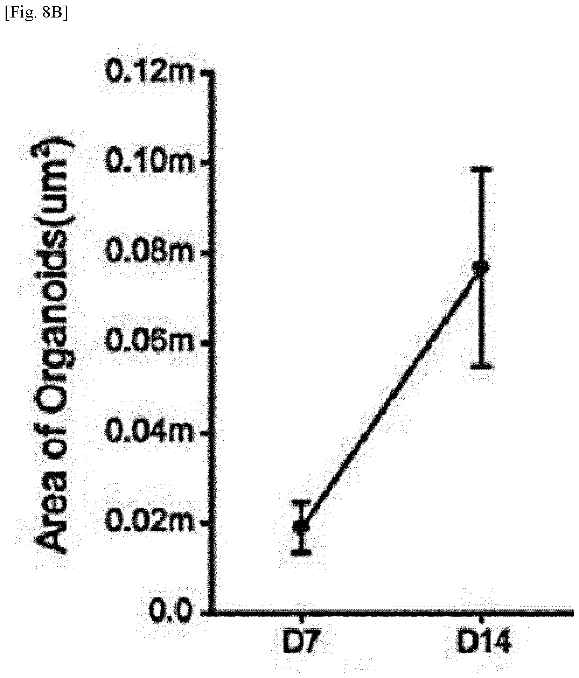

Moreover, the results are illustrated in FIGS. 7 and 8.

FIG 7A is a set of photographs illustrating the high-speed mass imaging results of Example 1-1, FIG 7B is a graph illustrating the area of the organoids cultured for a certain period of time in Example 1, FIG 8A is a set of photographs illustrating the high-speed mass imaging results of Comparative Example 1, and FIG 8B is a graph illustrating the area of the organoids cultured for a certain period of time in Comparative Example 1.

Referring to FIG 7, it can be confirmed that when the organoid prepared in Example 1-1 was subjected to automatic imaging, imaging can be performed without a large error because an imaging height is uniform, and due to this fact, an error range is very small when the actual area is measured.

In particular, when the organoid is cultured using the cell culture plate of the present invention, the organoid is cultured in a uniform size. That is, standardization is possible. As a result of standardization, the focus was automatically determined during image measurement, and a deviation for the measured height was minimized by a connector structure. Accordingly, when the screening image is measured, a deviation of around 20 µm is exhibited, which is very small.

Referring to FIG 8, it can be confirmed that in the case of Comparative Example 1, the organoids grow while overlapping each other, and it can be seen that the size and distribution position of the organoids are different, and thus cannot be standardized. Therefore, during the measurement of the screening image, a deviation of up to 150 µm is exhibited, which is large.

The results as described above are because when organoids are cultured by methods in the related art, since the organoids are grown randomly in Matrigel^{®}, it is difficult to uniformly culture a desired organoid, and a measured height is also variable, and thus, there is a limit in which it is difficult to apply the measured height to organoid screening imaging. Therefore, when the area of the organoid cultured for a certain period of time is analyzed, the deviation appears to be very large.

In the case of FIG 9, in the standard-type organoids produced as a whole, the diameter of each organoid was measured using the Image J program. A total of 864 wells were subjected to high-speed mass imaging, it was confirmed that a uniform diameter was obtained by analyzing each of the images by ImageJ.

FIG 9 illustrates the imaging results and the size of the organoid when the colorectal cancer cells of Example 1-1 were cultured for 14 days. It can be seen that it is possible to manufacture a standard organoid because the size of the organoids is uniform at 300 to 500 µm.

### Example B: Selection of cancer organoid having resistance to anticancer agent and radiation

### 1. Construction of colorectal cancer organoid

As previously shown, it was confirmed that the standard-type cancer organoid could be mass-produced using the cell culture plate of the present invention. Based on this, a cancer organoid having resistance to an anticancer agent and radiation was selected from a patient with cancer. Specifically, a human colorectal sample was obtained with the approval of the Institutional Review Board (IRB) of ASAN Medical Center, Seoul, Korea. This colorectal cancer tissue was made into cells and isolated into single cells, and then 10 types of uniform and standardized colorectal cancer organoids were constructed by including 2 vol% of Matrigel^{®} using the cell culture plate of the present invention (No. 1:F59, No. 2:M60, No. 3: F48, No. 4: F49, No. 5: F55, No. 6: F77, No. 7: M75, No. 8: M68, No. 9: M76, No. 10: M62).

### 2: Immunofluorescence staining method of cancer organoids

Cultured organoids were washed once with PBS, treated with a culture solution including 2 µM calcein AM, 2 µM EthD-1, and 2 drops/1 ml of Hoechst33342, and then treated in an incubator (37°C, CO₂ 5%) for 60 to 90 minutes. After the treatment, the culture solution was replaced with a culture solution which does not include a staining kit, and then the expression of cell nuclei, live cells, and dead cells was confirmed under a fluorescence microscope.

### 3. Measurement of viability of cancer organoid

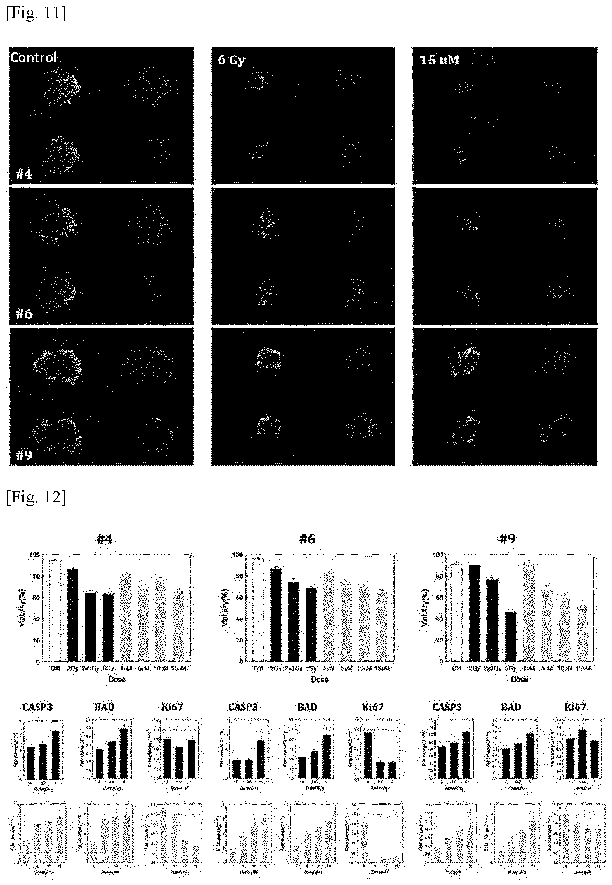

Based on the expression images of cell nuclei, live cells, and dead cells measured by the method in FIG 11, measurement was performed by ImageJ. After the image was processed with a threshold, an area ratio was measured. A viability of the organoid was obtained using the measured area ratio to calculate the ratio of live cells to total cells.

### 3: Selection of cancer organoid having resistance to anticancer agent and radiation

The above 10 types of colorectal cancer organoids were each independently treated with an anticancer agent (5-FU) and radiation. The colorectal cancer organoids were treated with the anticancer agent and radiation at a concentration of 1, 5, 10 and 15 µM and a concentration of 2, 2 + 2 + 2 and 6 Gy, respectively. A group untreated with the anticancer agent and the radiation was used as a control. As a result, as illustrated in FIG 10, No. 4, No. 6 and No. 9 were primarily selected. Referring to FIG 11, the size of the No. 9 cancer organoid among Nos. 4, 6 and 9 did not decrease significantly when treated with the anticancer agent and radiation. Therefore, the No. 9 cancer organoid was selected as an organoid having resistance to an anticancer agent and radiation.

### 4. Confirmation of resistance characteristics of selected organoids having resistance to radiation and anticancer agent

In FIG 12, PCR was performed to confirm the changes in gene units of organoids after drug treatment and irradiation with radiation. The organoids were transferred to a 1.5-ml tube and washed with PBS, and then RNA was extracted using an RNA extraction kit manufactured by Qiagen. The concentration of RNA was measured by NanoDrop^{™} (trade name), and based on this, RNA was converted into cDNA using a cDNA conversion kit manufactured by Applied Biosystems. In order to confirm the effects of drugs and radiation, primers capable of confirming the expression of caspase3 (CASP3) and BAD, which are representative genes related to cell death, and Ki67, which is related to cell proliferation were manufactured (Table 1), and gene expression of the organoids treated with the drug and radiation was confirmed by PCR based on primers and cDNA. Through this, it could be confirmed that in the case of organoids showing resistance to drugs and radiation, the expression of cell death was relatively low and the expression of cell proliferation was relatively high. Referring to FIG 12, it can be seen that in the case of the No. 9 cancer organoid, the expression of the cell proliferation gene is relatively higher than that of the cell death gene.

**[Table 1]**

| Genes | Primer sequences (5'-3') | |
|---|---|---|
| Caspase3 | forward | ggcgaaattcaaaggatggc |
| | reverse | aacccgggtaagaatgtgca |
| BAD | forward | tttgaggaccttcgaccagc |
| | reverse | aggtcttcagagtgagccca |
| Ki67 | forward | agctgactctgccactasgc |
| | reverse | gtccagctgtagtgcccaat |

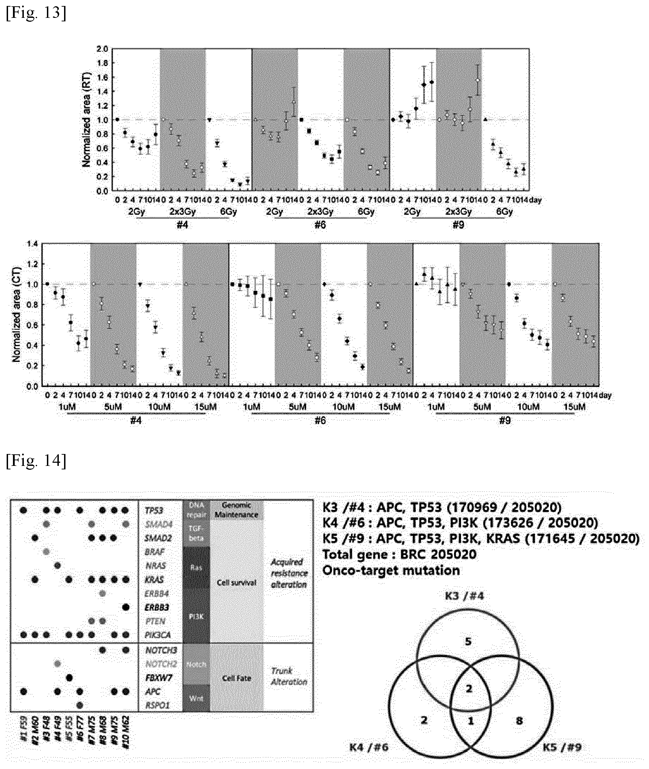

Figure 13 quantitatively illustrates the changes in size of organoids after drug and radiation treatment. The size of the organoid was measured using ImageJ based on an organoid phase photograph on each date. The corresponding graph shows the results of quantifying the organoid size of the entire experimental group by the method of standardizing the organoid size of the control to 1. It could be confirmed that in the case of the No. 9 organoid, the resistance to the anticancer agent and radiation was higher than those of the Nos. 4 and 6 organoids. Through this, it was confirmed that an organoid having resistance to a drug and radiation could be selected by only culturing the standard-type cancer organoid according to the present invention and measuring the size of the cultured cancer organoid.

Through FIG 13, a cancer organoid having resistance to radiation and an anticancer agent may be selected through its viability and size.

Specifically, referring to the data of No. 9, which is a group having resistance to radiation, the case of being irradiated with radiation at a low concentration and a low concentration of radiation several times (this case is similar to a general protocol/clinical trial) and the case of being irradiated with excessive radiation were compared with each other. In the case of a No. 9 patient (KRAS mutation patient/resistant to radiation and an anticancer agent), it can be seen that when a change in size for 7 days of being irradiated with radiation is observed, the size is maintained at an almost 70% to 100% level, the viability itself was not changed, and it can be seen that for 7 days of treatment, the size of cells is changed by an approximate 30% level. It can be confirmed that the cells proliferate and increase in size rapidly without treatment after 7 days.

It could be seen that a No. 9 patent (KRAS mutation patient/resistant to radiation and an anticancer agent), which is a group having resistance to an anticancer agent did not exhibit any response to a drug at a low concentration, the viability ranges from 100% to 70%, and the morphology of cells was not reduced. In addition, while the KI67 gene increased rapidly even after the drug was removed, a pattern showing the appearance of suppressing cell death was exhibited. These results are a phenomenon typical of a patient having resistance to an anticancer agent, where viability is desired to be maintained within a range of 70%, and it could be confirmed that while the cell proliferation rate became more uniform than the cell death rate, the cells rapidly recovered in the future. Furthermore, it was confirmed that even in the change in size, when the size of the period during which the drug was administered for the initial 7 days was confirmed, the case where the size is not reduced by 60% or more showed a pattern in which cells recovered again.

FIG 14 summarizes standard-type cancer organoids produced according to the present invention by main mutation characteristics by categorizing the cancer organoids by each mutation and grouping the cancer organoids by characteristics of cells.

As an experimental method, the organoids were transferred to a 1.5-ml tube and washed with PBS, and then RNA was extracted using an RNA extraction kit manufactured by Qiagen. Genetic information on RNA was confirmed based on a TruSeq RNAAccess library provided by Illumina.

Referring to FIG 14, it could be confirmed that as a result of genomic tests of specimens selected after drug and irradiation with radiation, complex oncotarget mutagenesis such as TP53, APC, PIK3CA and KRAS was exacerbated in specimens showing resistance. In particular, it could be confirmed that in the case of the No. 9 organoid, all of TP53, APC, PIK3CA and KRAS were expressed. Through this, it can be seen that when the cancer organoid having resistance to an anticancer agent and/or radiation selected by the present invention is used, a patient having resistance to an anticancer agent and/or radiation can be diagnosed without a separate genomic test.

A drug which alleviates the drug resistance of cancer cells can be screened by treating the thus selected organoid having resistance to an anticancer agent and/or radiation with a candidate material which alleviates the drug resistance of cancer cells along with a cancer resistance drug. Specifically, when a candidate drug which alleviates the drug resistance of cancer cells is effective, the viability of the organoid having resistance to an anticancer agent and/or radiation selected by the present invention will be lower than that of the control (group untreated with the candidate drug which alleviates the drug resistance of cancer cells).

Further, when the present invention is used, a cancer tissue is isolated from a patient with cancer and cultured in the 3D cell culture plate mentioned in the present invention to produce a standard-type cancer organoid, and then it can be easily diagnosed whether the cancer organoid has resistance to an anticancer agent and radiation. Moreover, if the patient is diagnosed with a cancer patient having resistance to an anticancer agent and/or radiation, a drug which alleviates the resistance of cancer cells can be screened by treating this cancer organoid with a material which alleviates the drug resistance of cancer cells. That is, patient-customized drug screening is possible.

As described above, through the standard-type organoid production method of the present invention, an organoid having resistance to an anticancer agent and/or radiation is selected and confirmed only with the steps of culturing the organoid and treating the organoid with an anticancer agent and/or a drug without a complex genomic test by culturing a cancer tissue obtained from a patient with cancer into organoids, and through this, it can be diagnosed whether the patient with cancer has resistance to an anticancer agent and/or radiation.

Although a specific part of the present invention has been described in detail, it will be obvious to those skilled in the art that such a specific description is just a preferred embodiment and the scope of the present invention is not limited thereby. Accordingly, the substantial scope of the present invention will be defined by the appended claims and equivalents thereof.

### [Description of Reference Numerals and Symbols]

100: Well plate
101: Step
110: Main well
120: Sub well
121: Recessed part
130: Space part
140: Concave part
200: Connector for large-capacity and high-speed HCS
210: Base
220: Cover
240: Convex part

## Claims

1. A method for diagnosis of resistance to an anticancer agent and/or radiation of a subject with cancer, the method including:
providing an isolated cancer tissue from a subject with cancer;
culturing the cancer tissue into a cancer organoid in a 3D cell culture plate (10):
treating the cultured cancer organoid with an anticancer agent and/or radiation; and
selecting the cancer organoid treated with the anticancer agent and/or radiation as a cancer organoid having resistance to an anticancer agent and/or radiation when the cancer organoid treated with the anticancer agent and/or radiation has a viability equal to or more than that before the treatment with the anticancer agent and/or radiation,
wherein in the culturing of the organoid, the 3D cell culture plate (10) comprises 0 to 2 vol% of an extracellular matrix-based hydrogel, and
wherein the 3D cell culture plate (10) comprises:
a well plate (100) comprising a plurality of main wells (110) and a plurality of sub wells (120) formed at lower portions of the main wells (110) to be injected with a cell culture solution and comprising recessed parts (121) on a bottom surface thereof; and a connector for large-capacity and high-speed high content screening (HCS) (200), which supports the well plate (100), and
the connector for high content screening (HCS) (200) comprises a base (210) equipped with a fixing means so as to be attached to and detached from a lower end of the well plate (100) and a cover (220) positioned on an upper portion of the well plate (100) to be coupled to the base (210), the main well (110) has a step (101) formed so as to be tapered at a predetermined site, and the step (101) has an inclination angle (θ) ranging from 10 to 60° with respect to a wall of the main well (110).

2. The method of claim 1, wherein in the selecting of the cancer organoid treated with the anticancer agent and/or radiation as a cancer organoid having resistance to an anticancer agent and/or radiation, the cancer organoid is selected as a cancer organoid having resistance to an anticancer agent and/or radiation when the cancer organoid treated with the anticancer agent and/or radiation has a viability ranging from 100 to 70% compared to an organoid before treatment with an anticancer agent and/or radiation.

3. The method of claim 1, wherein in the selecting of the cancer organoid treated with the anticancer agent and/or radiation as a cancer organoid having resistance to an anticancer agent and/or radiation, the cancer organoid is selected as a cancer organoid having resistance to an anticancer agent and/or radiation when the cancer organoid treated with the anticancer agent and/or radiation has a size which is not reduced by 30% to 60% or more compared to an organoid before treatment with an anticancer agent and/or radiation.

4. The method of claim 1, wherein the extracellular matrix-based hydrogel is Matrigel^{®}.

5. The method of claim 1, wherein a size of the cancer organoids is 300 to 500 µm in diameter.

6. The method of claim 1, wherein the cancer is colorectal cancer, lung cancer, gastric cancer, skin cancer, prostate cancer, breast cancer, cervical cancer, thyroid cancer, fibrosarcoma, uterine sarcoma, or hematological cancer.

7. The method of claim 1, wherein the anticancer agent is one or more anticancer agents selected from the group consisting of a cytotoxic anticancer agent, an immune anticancer agent, a targeted anticancer therapeutic, and a metabolic anticancer agent.

8. The method of claim 1, wherein the sub well (120) has an inclined surface formed so as to taper toward the recessed part (121),
the sub wells (120) have an upper end diameter ranging from 3.0 to 4.5 mm,
the recessed parts (121) have an upper end diameter ranging from 0.45 to 1.5 mm,
an inclined surface (θ₂) between the sub well (120) and the recessed part (121) ranges from 40 to 50°, and
a length ratio of the diameter of the sub wells (120) to the diameter of the recessed parts (121) ranges from 1:0.1 to 0.5.

9. The method of claim 1, wherein the main well (110) has an individual volume ranging from 100 to 300 µl,
the recessed part (121) has an individual volume ranging from 20 to 50 µl, and
an individual volume ratio of the main well (110) to the recessed part (121) is 1:0.1 to 0.5 on average.

10. The method of claim 1, wherein the main well (110) comprises a space part (130) between the step (101) and the sub well (120),
the space part (130) has a height (aₕ) ranging from 2.0 to 3.0 mm on average,
the sub well (120) has a height (bₕ) ranging from 1.0 to 2.0 mm on average, and
a height ratio (aₕ:bₕ) of the space part (130) to the sub well (120) ranges from 1:0.3 to 1.

11. A method of screening a drug which alleviates the drug resistance of cancer cells, the method comprising:
providing an isolated cancer tissue from a subject with cancer;
culturing the cancer tissue into a cancer organoid in a 3D cell culture plate (10):
treating the cultured cancer organoid with an anticancer agent and/or radiation; and
selecting the cancer organoid treated with the anticancer agent and/or radiation as a cancer organoid having resistance to an anticancer agent and/or radiation when the cancer organoid treated with the anticancer agent and/or radiation has a viability equal to or more than that before the treatment with the anticancer agent and/or radiation
treating the organoid selected as the cancer organoid having resistance to the anticancer agent and/or radiation with a candidate material which alleviates the drug resistance of cancer cells along with a cancer resistance drug;
comparing a cancer organoid viability of a group treated with the candidate material with a cancer organoid viability of a control untreated with the candidate material; and
determining the candidate material as a drug for alleviating the drug resistance of cancer cells when the cancer organoid viability of the group treated with the candidate material is lower than the viability of the control,
wherein in the culturing of the organoid, the 3D cell culture plate (10) comprises 0 to 2 vol% of an extracellular matrix-based hydrogel, and
wherein the 3D cell culture plate (10) comprises:
a well plate (100) comprising a plurality of main wells (110) and a plurality of sub wells (120) formed at lower portions of the main wells (110) to be injected with a cell culture solution and comprising recessed parts (121) on a bottom surface thereof; and a connector for large-capacity and high-speed high content screening (HCS) (200), which supports the well plate (100), and
the connector for high content screening (HCS) (200) comprises a base (210) equipped with a fixing means so as to be attached to and detached from a lower end of the well plate (100) and a cover (220) positioned on an upper portion of the well plate (100) to be coupled to the base(210), the main well (110) has a step (101) formed so as to be tapered at a predetermined site, and the step (101) has an inclination angle (θ) ranging from 10 to 60° with respect to a wall of the main well (110).

## Patentansprüche

1. Verfahren für eine Diagnose einer Resistenz gegenüber einem Antikrebsmittel und/oder einer Strahlung eines Subjekts mit Krebs, wobei das Verfahren umfasst:
Vorsehen eines isolierten Krebsgewebes von einem Subjekt mit Krebs,
Kultivieren des Krebsgewebes zu einem Krebs-Organoid in einer 3D-Zellkulturplatte (10),
Behandeln des kultivierten Krebs-Organoids mit einem Antikrebsmittel und/oder einer Strahlung, und
Auswählen des mit dem Antikrebsmittel und/oder der Strahlung behandelten Krebs-Organoids als eines Krebs-Organoids mit einer Resistenz gegenüber einem Antikrebsmittel und/oder einer Strahlung, wenn das mit dem Antikrebsmittel und/oder der Strahlung behandelte Krebs-Organoid eine Lebensfähigkeit aufweist, die gleich oder größer als diejenige vor der Behandlung mit dem Antikrebsmittel und/oder der Strahlung ist,
wobei bei dem Kultivieren des Organoids die 3D-Zellkulturplatte (10) 0 bis 2 Vol-% eines extrazellulären Matrix-basierten Hydrogels enthält, und
wobei die 3D-Zellkulturplatte (10) umfasst:
eine Well-Platte (100), die eine Vielzahl von Haupt-Wells (110) und eine Vielzahl von an unteren Teilen der Haupt-Wells (110) ausgebildeten Neben-Wells (120), in die eine Zellkulturlösung injiziert wird und die vertiefte Teile (121) an einer unteren Fläche aufweisen, umfasst, und
ein Verbindungsglied (200) für ein High-Content-Screening (HCS) mit großer Kapazität und hoher Geschwindigkeit, das die Weil-Platte (100) hält,
wobei das Verbindungsglied (200) für ein High-Content-Screening (HCS) eine Basis (210), die mit einer Fixierungseinrichtung für eine Anbringung und Lösung an und von einem unteren Ende der Well-Platte (100) versehen ist, und eine Abdeckung (220), die an einem oberen Teil der Well-Platte (100) für eine Kopplung mit der Basis (210) angeordnet ist, umfasst, wobei das Haupt-Well (110) eine Stufe (101) für eine Verjüngung an einer vorbestimmten Position aufweist und die Stufe (101) einen Neigungswinkel (θ) im Bereich von 10 bis 60° in Bezug auf eine Wand des Haupt-Wells (110) aufweist.

2. Verfahren nach Anspruch 1, wobei beim Auswählen des mit dem Antikrebsmittel und/oder der Strahlung behandelten Krebs-Organoids als eines Krebs-Organoids mit einer Resistenz gegenüber einem Antikrebsmittel und/oder einer Strahlung das Krebs-Organoid als ein Krebs-Organoid mit einer Resistenz gegenüber einem Antikrebsmittel und/oder einer Strahlung ausgewählt wird, wenn das mit dem Antikrebsmittel und/oder der Strahlung behandelte Krebs-Organoid eine Lebensfähigkeit im Bereich von 100 bis 70% im Vergleich zu einem Organoid vor einer Behandlung mit einem Antikrebsmittel und/oder einer Strahlung aufweist.

3. Verfahren nach Anspruch 1, wobei beim Auswählen des mit dem Antikrebsmittel und/oder der Strahlung behandelten Krebs-Organoids als eines Krebs-Organoids mit einer Resistenz gegenüber einem Antikrebsmittel und/oder einer Strahlung das Krebs-Organoid als ein Krebs-Organoid mit einer Resistenz gegenüber einem Antikrebsmittel und/oder einer Strahlung ausgewählt wird, wenn das mit dem Antikrebsmittel und/oder der Strahlung behandelte Krebs-Organoid eine Größe aufweist, die nicht um 30% bis 60% oder mehr im Vergleich zu einem Organoid vor einer Behandlung mit einem Antikrebsmittel und/oder einer Strahlung reduziert ist.

4. Verfahren nach Anspruch 1, wobei das extrazelluläre Matrix-basierte Hydrogel Matrigel^{®} ist.

5. Verfahren nach Anspruch 1, wobei der Durchmesser der Krebs-Organoide von 300 bis 500 µm beträgt.

6. Verfahren nach Anspruch 1, wobei der Krebs ein Dickdarmkrebs, Lungenkrebs, Magenkrebs, Hautkrebs, Prostatakrebs, Brustkrebs, Gebärmutterhalskrebs, Schilddrüsenkrebs, Fibrosarkom, Uterussarkom oder Blutkrebs ist.

7. Verfahren nach Anspruch 1, wobei das Antikrebsmittel eines oder mehrere Antikrebsmittel ist, die aus der Gruppe ausgewählt sind, die aus einem zytotoxischen Antikrebsmittel, einem immuntherapeutischen Antikrebsmittel, einem gezielten therapeutischen Mittel und einem metabolischen Antikrebsmittel besteht.

8. Verfahren nach Anspruch 1, wobei das Neben-Well (120) eine geneigte Fläche aufweist, die derart ausgebildet ist, dass sie sich zu dem vertieften Teil (121) verjüngt,
wobei die Neben-Wells (120) einen oberen Enddurchmesser im Bereich von 3,0 bis 4,5 mm aufweisen,
wobei die vertieften Teile (121) einen oberen Enddurchmesser im Bereich von 0,45 bis 1,5 mm aufweisen,
wobei die Neigung einer geneigten Fläche (θ₂) zwischen dem Neben-Well (120) und dem vertieften Teil (121) im Bereich von 40 bis 50° liegt, und
wobei das Längenverhältnis zwischen dem Durchmesser der Neben-Wells (120) und dem Durchmesser der vertieften Teile (121) im Bereich von 1:0,1 bis 0,5 liegt.

9. Verfahren nach Anspruch 1, wobei das Haupt-Well (110) ein individuelles Volumen im Bereich von 100 bis 300 µl aufweist,
wobei der vertiefte Teil (121) ein individuelles Volumen im Bereich von 20 bis 50 µl aufweist, und
wobei das Verhältnis der individuellen Volumen des Haupt-Wells (110) und des vertieften Teils (121) durchschnittlich 1:0,1 bis 05 beträgt.

10. Verfahren nach Anspruch 1, wobei das Haupt-Well (110) einen Raumteil (130) zwischen der Stufe (101) und dem Neben-Well (120) aufweist,
wobei der Raumteil (130) eine Höhe (aₕ) im Bereich von durchschnittlich 2,0 bis 3,0 mm aufweist,
wobei das Neben-Well (120) eine Höhe (bₕ) im Bereich von durchschnittlich 1,0 bis 2,0 mm aufweist, und
wobei das Höhenverhältnis (aₕ:bₕ) des Raumteils (130) zu dem Neben-Well (120) im Bereich von 1:0,3 bis 1 liegt.

11. Verfahren für ein Screening eines Arzneimittels, das die Arzneimittelresistenz von Krebszellen vermindert, wobei das Verfahren umfasst:
Vorsehen eines isolierten Krebsgewebes von einem Subjekt mit Krebs,
Kultivieren des Krebsgewebes zu einem Krebs-Organoid in einer 3D-Zellkulturplatte (10),
Behandeln des kultivierten Krebs-Organoids mit einem Antikrebsmittel und/oder einer Strahlung, und
Auswählen des mit dem Antikrebsmittel und/oder der Strahlung behandelten Krebs-Organoids als eines Krebs-Organoids mit einer Resistenz gegenüber einem **Antikrebsmittel** und/oder einer Strahlung, wenn das mit dem Antikrebsmittel und/oder der Strahlung behandelte Krebs-Organoid eine Lebensfähigkeit aufweist, die gleich oder größer als diejenige vor der Behandlung mit dem Antikrebsmittel und/oder der Strahlung ist,
Behandeln des als des Krebs-Organoids mit einer Resistenz gegenüber dem Antikrebsmittel und/oder der Strahlung ausgewählten Organoids mit einem Kandidatenmittel, das die Arzneimittel-Resistenz von Krebszellen vermindert, und mit einem Krebsresistenz-Arzneimittel,
Vergleichen der Krebs-Organoid-Lebensfähigkeit einer mit dem Kandidatenmaterial behandelten Gruppe mit der Krebs-Organoid-Lebensfähigkeit einer nicht mit dem Kandidatenmaterial behandelten Kontrolle, und
Bestimmen des Kandidaten-Materials als eines Arzneimittels für das Vermindern der Arzneimittel-Resistenz von Krebszellen, wenn die Krebs-Organoid-Lebensfähigkeit der mit dem Kandidatenmaterial behandelten Gruppe niedriger als die Lebensfähigkeit der Kontrolle ist,
wobei beim Kultivieren des Organoids die 3D-Zellkulturplatte (10) 0 bis 2 Vol.-% eines extrazellulären Matrix-basierten Hydrogels enthält, und
wobei die 3D-Zellkulturplatte (10) umfasst:
eine Well-Platte (100), die eine Vielzahl von Haupt-Wells (110) und eine Vielzahl von an unteren Teilen der Haupt-Wells (110) ausgebildeten Neben-Wells (120), in die eine Zellkulturlösung injiziert wird und die vertiefte Teile (121) an einer unteren Fläche aufweisen, umfasst, und
ein Verbindungsglied (200) für ein High-Content-Screening (HCS) mit großer Kapazität und hoher Geschwindigkeit, das die Well-Platte (100) hält,
wobei das Verbindungsglied (200) für ein High-Content-Screening (HCS) eine Basis (210), die mit einer Fixierungseinrichtung für eine Anbringung und Lösung an und von einem unteren Ende der Well-Platte (100) versehen ist, und eine Abdeckung (220), die an einem oberen Teil der Well-Platte (100) für eine Kopplung mit der Basis (210) angeordnet ist, umfasst, wobei das Haupt-Well (110) eine Stufe (101) für eine Verjüngung an einer vorbestimmten Position aufweist und die Stufe (101) einen Neigungswinkel (θ) im Bereich von 10 bis 60° in Bezug auf eine Wand des Haupt-Wells (110) aufweist.

## Revendications

1. Procédé de diagnostic de la résistance à un agent anticancéreux et/ou à un rayonnement d'un sujet atteint d'un cancer, le procédé incluant :
la fourniture d'un tissu cancéreux isolé
provenant d'un sujet atteint d'un cancer ;
la culture du tissu cancéreux en un organoïde cancéreux dans une plaque de culture cellulaire 3D (10) :
le traitement de l'organoïde cancéreux cultivé par un agent anticancéreux et/ou un rayonnement ; et
la sélection de l'organoïde cancéreux traité par l'agent anticancéreux et/ou le rayonnement en tant qu'organoïde cancéreux présentant une résistance à un agent anticancéreux et/ou à un rayonnement lorsque l'organoïde cancéreux traité par l'agent anticancéreux et/ou le rayonnement présente une viabilité égale ou supérieure à celle qui existait avant le traitement par l'agent anticancéreux et/ou le rayonnement,
dans lequel, lors de la culture de l'organoïde, la plaque de culture cellulaire 3D (10) comprend 0 à 2 % en volume d'un hydrogel à base de matrice extracellulaire, et
dans lequel la plaque de culture cellulaire 3D (10) comprend :
une plaque (100) à puits comprenant une pluralité de puits principaux (110) et une pluralité de sous-puits (120) formés dans les parties inférieures des puits principaux (110), destinés à recevoir l'injection d'une solution de culture cellulaire et comprenant des parties en retrait (121) sur une surface inférieure de ceux-ci ; et un connecteur pour le criblage à haut contenu (HCS) à grande capacité et à grande vitesse (200), qui supporte la plaque (100) à puits, et
le connecteur pour le criblage à haut contenu (HCS) (200) comprend une base (210) équipée d'un moyen de fixation pour être attachée à et détachée d'une extrémité inférieure de la plaque (100) à puits et un couvercle (220) positionné sur une partie supérieure de la plaque (100) à puits pour être couplé à la base (210), le puits principal (110) a un gradin (101) formé de manière à être rétréci à un endroit prédéterminé, et le gradin (101) a un angle d'inclinaison (θ) se situant dans la plage de 10 à 60° par rapport à une paroi du puits principal (110).

2. Procédé selon la revendication 1, dans lequel lors de la sélection de l'organoïde cancéreux traité par l'agent anticancéreux et/ou le rayonnement en tant qu'organoïde cancéreux ayant une résistance à un agent anticancéreux et/ou à un rayonnement, l'organoïde cancéreux est sélectionné en tant qu'organoïde cancéreux ayant une résistance à un agent anticancéreux et/ou à un rayonnement lorsque l'organoïde cancéreux traité par l'agent anticancéreux et/ou le rayonnement a une viabilité se situant dans la plage de 100 à 70 % par rapport à un organoïde avant le traitement par un agent anticancéreux et/ou un rayonnement.

3. Procédé selon la revendication 1, dans lequel, lors de la sélection de l'organoïde cancéreux traité par l'agent anticancéreux et/ou le rayonnement en tant qu'organoïde cancéreux résistant à un agent anticancéreux et/ou à un rayonnement, l'organoïde cancéreux est sélectionné en tant qu'organoïde cancéreux résistant à un agent anticancéreux et/ou à un rayonnement lorsque l'organoïde cancéreux traité par l'agent anticancéreux et/ou le rayonnement a une taille qui n'est pas réduite de 30 % à 60 % ou plus par rapport à un organoïde avant le traitement par un agent anticancéreux et/ou un rayonnement.

4. Procédé selon la revendication 1, dans lequel l'hydrogel à base de matrice extracellulaire est Matrigel^{®}.

5. Procédé selon la revendication 1, dans lequel une taille des organoïdes cancéreux est de 300 à 500 µm en diamètre.

6. Procédé selon la revendication 1, dans lequel le cancer est un cancer colorectal, un cancer du poumon, un cancer gastrique, un cancer de la peau, un cancer de la prostate, un cancer du sein, un cancer du col de l'utérus, un cancer de la thyroïde, un fibrosarcome, un sarcome utérin ou un cancer hématologique.

7. Procédé selon la revendication 1, dans lequel l'agent anticancéreux est un ou plusieurs agents anticancéreux choisis dans le groupe constitué par un agent anticancéreux cytotoxique, un agent anticancéreux immunothérapeutique, un agent thérapeutique anticancéreux ciblé et un agent anticancéreux métabolique.

8. Procédé selon la revendication 1, selon lequel le sous-puits (120) a une surface inclinée formée de manière à se rétrécit vers la partie en retrait (121),
les sous-puits (120) ont un diamètre d'extrémité supérieur se situant dans la plage de 3,0 à 4,5 mm,
les parties en retrait (121) ont un diamètre d'extrémité supérieure valant de 0,45 à 1,5 mm,
une surface inclinée (02) entre le sous-puits (120) et la partie en retrait (121) se situe dans la plage de 40 à 50°, et
un rapport de longueur entre le diamètre des sous-puits (120) et le diamètre des parties en retrait (121) se situe dans la plage de 1:0,1 à 0,5.

9. Procédé selon la revendication 1, dans lequel le puits principal (110) a un volume individuel se situant dans la plage de 100 à 300 µl,
la partie en retrait (121) a un volume individuel se situant dans la plage de 20 à 50 µl, et
un rapport de volume individuel entre le puits principal (110) et la partie en retrait (121) est de 1:0,1 à 0,5 en moyenne.

10. Procédé selon la revendication 1, dans lequel le puits principal (110) comprend une partie espace (130) entre le gradin (101) et le sous-puits (120),
la partie espace (130) a une hauteur (aₕ) se situant dans la plage de 2,0 à 3,0 mm en moyenne,
le sous-puits (120) a une hauteur (bₕ) se situant dans la plage de 1,0 à 2,0 mm en moyenne, et
un rapport de hauteur (aₕ:bₕ) entre la partie espace (130) et le sous-puits (120) se situe dans la plage de 1:0,3 à 1.

11. Procédé de criblage d'un médicament qui atténue la résistance aux médicaments de cellules cancéreuses, le procédé comprenant :
la fourniture d'un tissu cancéreux isolé provenant d'un sujet atteint d'un cancer ;
la culture du tissu cancéreux en un organoïde cancéreux dans une plaque de culture cellulaire 3D (10) :
le traitement de l'organoïde cancéreux cultivé par un agent anticancéreux et/ou un rayonnement ; et
la sélection de l'organoïde cancéreux traité par l'agent anticancéreux et/ou le rayonnement en tant qu'organoïde cancéreux présentant une résistance à un agent anticancéreux et/ou à un rayonnement lorsque l'organoïde cancéreux traité par l'agent anticancéreux et/ou le rayonnement présente une viabilité égale ou supérieure à celle qui existait avant le traitement par l'agent anticancéreux et/ou le rayonnement
le traitement de l'organoïde sélectionné en tant que l'organoïde cancéreux résistant à l'agent anticancéreux et/ou au rayonnement par une substance candidate qui atténue la résistance des cellules cancéreuses aux médicaments, ainsi que par un médicament anticancéreux ;
la comparaison d'une viabilité d'un organoïde cancéreux d'un groupe traité par la substance candidate à une viabilité d'un organoïde cancéreux d'un groupe témoin non traité par la substance candidate ; et
la détermination de la substance candidats en tant qu'un médicament pour atténuer la résistance aux médicaments de cellules cancéreuses lorsque la viabilité d'organoïde cancéreux du groupe traité par la substance candidate est inférieure à la viabilité du témoin,
dans lequel, lors de la culture de l'organoïde, la plaque de culture cellulaire 3D (10) comprend 0 à 2 % en volume d'un hydrogel à base de matrice extracellulaire, et
dans lequel la plaque de culture cellulaire 3D (10) comprend :
une plaque (100) à puits comprenant une pluralité de puits principaux (110) et une pluralité de sous-puits (120) formés dans les parties inférieures des puits principaux (110), destinés à recevoir l'injection d'une solution de culture cellulaire et comprenant des parties en retrait (121) sur une surface inférieure de ceux-ci ; et un connecteur pour le criblage à haut contenu (HCS) à grande capacité et à grande vitesse (200), qui supporte la plaque (100) à puits, et
le connecteur pour le criblage à haut contenu (HCS) (200) comprend une base (210) équipée d'un moyen de fixation pour être attachée à et détachée d'une extrémité inférieure de la plaque (100) à puits et un couvercle (220) positionné sur une partie supérieure de la plaque (100) à puits pour être couplé à la base (210), le puits principal (110) a un gradin (101) formé de manière à être rétréci à un endroit prédéterminé, et le gradin (101) a un angle d'inclinaison (θ) se situant dans la plage de 10 à 60° par rapport à une paroi du puits principal (110).
